Europäisches Patentamt

European Patent Office

Office européen des brevets

(11) Numéro de publication: **0 402 244 B1**

(12) **FASCICULE DE BREVET EUROPEEN**

(45) Date de publication du fascicule du brevet: **25.10.95**  (51) Int. Cl.6: **A61B 6/00**, G01N 23/04, G01N 23/08

(21) Numéro de dépôt: **90401529.4**

(22) Date de dépôt: **06.06.90**

(54) Procédé d'étalonnage d'un système radiologique et de mesure de l'épaisseur équivalente d'un objet.

(30) Priorité: **09.06.89 FR 8907686**

(43) Date de publication de la demande:
**12.12.90 Bulletin 90/50**

(45) Mention de la délivrance du brevet:
**25.10.95 Bulletin 95/43**

(84) Etats contractants désignés:
**DE GB NL**

(56) Documents cités:
**GB-A- 2 004 437**
**US-A- 4 763 343**

**MEDICAL PHYSICS, vol. 15, no. 6, décembre 1988, pages 904-908, New York, US; R. A. GEISE et al.: "Routine quality control tests for film-screen mammographic systems with automatic exposure control"**

**APPLIED OPTICS, vol. 23, no. 6, 1 mars 1984, pages 762-766; D. PASINI et al.: "In-situ calibration technique for X-ray films"**

(73) Titulaire: **GENERAL ELECTRIC CGR S.A.**
**100, rue Camille-Desmoulins**
**F-92130 Issy les Moulineaux (FR)**

(72) Inventeur: **Heidsieck, Robert**
**Cabinet Ballot-Schmit,**
**7, rue Le Sueur**
**F-75116 Paris (FR)**

(74) Mandataire: **Ballot, Paul Denis Jacques et al**
**Cabinet Ballot-Schmit,**
**7, rue Le Sueur**
**F-75116 Paris (FR)**

## Description

L'invention concerne les systèmes de radiologie pour examiner un objet et, plus particulièrement dans de tels systèmes, un procédé qui permet d'étalonner un système radiologique et de mesurer l'épaisseur équivalente de l'objet à examiner.

Un système de radiologie comprend essentiellement un tube à rayons X et un détecteur d'un tel rayonnement entre lesquels on interpose l'objet à examiner tel qu'une partie du corps d'un patient. Le détecteur, qui est par exemple un couple film-écran, fournit après une durée de pose appropriée et développement du film, une image de l'objet. La qualité de cette image dépend à la fois des caractéristiques de cet objet et des paramètres du système de radiologie.

Les propriétés radiologiques de l'objet sont données par son épaisseur et sa composition et varient, d'une part, d'un patient à l'autre, et, d'autre part, selon la partie du corps à examiner. Or, il est difficile d'appréhender avec précision ces caractéristiques; en particulier, il est délicat, voire impossible, de déterminer la composition d'un objet par un seul examen physique. La notion d'épaisseur équivalente, connue en radiologie, permet de réduire la connaissance de ces deux variables à un problème à une dimension.

Un procédé d'étalonnage d'un système de radiologie est connu du document GB-A-2 004 437, ce procédé comprenant principalement les opérations suivantes: premièrement le choix d'étalons d'épaisseur connue et différente qui sont placés sur le trajet du rayonnement X, ensuite, pour des valeurs connues de la tension d'alimentation du tube à rayons X les différentes valeurs des signaux d'un détecteur sont obtenues, d'où résulte une table de valeurs de laquelle on peut tirer l'épaisseur équivalente d'un objet à examiner.

L'épaisseur équivalente d'un objet est définie par rapport à un matériau de référence tel que du plexiglass ou un matériau simulant l'absorption d'un organe d'une composition donnée. Dans des conditions radiologiques précises, c'est-à-dire configuration et paramètres de pose fixés, l'épaisseur équivalente d'un objet placé dans le champ de radiation est représentée par l'épaisseur de matériau de référence fournissant la même quantité d'énergie au détecteur, c'est-à-dire la même densité optique dans le cas d'un film.

Il est possible pour le médecin d'utiliser la connaissance de l'épaisseur équivalente de l'objet, par exemple, comme indication médicale ou pour réaliser des statistiques sur ces patients.

L'épaisseur équivalente de l'objet dépend également des paramètres du système radiologique. Ces paramètres sont généralement classés en deux catégories :

- les paramètres dits radiologiques tels que la tension V du tube à rayons X, le courant I de ce tube, le temps de pose S et le produit I x S qui définit la quantité d'énergie qui est émise,
- les paramètres dits de configuration qui sont tous les paramètres autres que radiologiques influant sur la qualité du rayonnement incident sur le détecteur, à l'exception de l'objet.

Ces paramètres de configuration sont par exemple :
a) le choix de la piste de l'anode tournante du tube à rayons X,
b) le choix de la taille du foyer du tube à rayons X,
c) le choix d'un filtre à interposer sur le trajet du faisceau de rayons X,
d) le choix du grandissement,
e) le choix de la distance entre le foyer et le récepteur d'image,
f) le choix du récepteur d'image,
g) le choix du type d'accessoires qui sera présent dans le faisceau de rayons X, par exemple, la pelote de compression, la grille anti-diffusante, etc...

Le but de la présente invention est de mettre en oeuvre un procédé d'étalonnage d'un système de radiologie qui permet de déterminer des relations entre les paramètres radiologiques du système, l'objet à radiographier et une grandeur caractéristique du spectre irradiant ayant traversé cet objet.

Le but de la présente invention est également de mettre en oeuvre un procédé qui permette de mesurer l'épaisseur équivalente d'un objet.

L'invention se rapporte à un procédé d'étalonnage d'un système de radiologie prévu pour examiner un objet, ledit système comprenant un tube à rayons X dont la tension d'alimentation V peut prendre diverses valeurs $V_m$, à variation continue ou discrète, et qui émet un faisceau de rayons X sous forme d'impulsions de durée variable S, et une seule cellule détectrice des rayons X ayant traversé l'objet à examiner qui permet de convertir une grandeur physique caractérisant le faisceau de rayons X en un signal de mesure M tel qu'un signal électrique,

le procédé comprenant les opérations suivantes :
(a) le choix d'une grandeur physique A qui caractérise l'objet à observer,
(b) le choix d'une classe d'objets de référence qui comporte n objets ou étalons dont la grandeur physique A peut prendre n valeurs connues $A_p$,

2

(c) le choix de j valeurs $V_m$ de la tension d'alimentation du tube à rayons X pour lesquelles l'étalonnage sera effectué,

(d) le choix de la valeur du produit IxS du courant anodique I débité par le tube à rayons X pendant la durée S de la pose pour chaque étalon associé à chaque valeur de la tension $V_m$ ,

(e) la mise en place d'un étalon sur le trajet du rayonnement X, le réglage de la tension du tube à une valeur $V_m$ et la mesure intégrée M du rayonnement ayant traversé l'étalon, qui a été détecté par la cellule détectrice entre le début de la mesure et l'instant où le produit IxS est égal à la valeur choisie pendant l'opération (d),

(f) le calcul du rendement D donné par le rapport M/IxS,

(g) la réitération des opérations (e) et (f) pour le même étalon mais pour les (j-1) autres valeurs de la tension d'alimentation $V_m$,

(h) la réitération des opérations (e), (f) et (g) pour les (n-1) autres objets ou étalons,

(i) la détermination du modèle analytique $D_i = f(V_m, A_p)$ reliant les (nxj) valeurs du rendement $D_i$ à celles de la grandeur physique $A_p$ et de la tension $V_m$ et,

(j) la détermination de la fonction inverse de $f(V_m, A_p)$ notée $g(V_m, D_i)$, qui permet de déterminer A en connaissant $V_m$ et $D_i$.

Les opérations (d) et (e) peuvent être remplacées par les opérations suivantes :

(d') le choix des temps de pose S pour chaque étalon associé à chaque valeur de la tension $V_m$ ,

(e') la mise en place d'un étalon sur le trajet du rayonnement X, le réglage de la tension du tube à une valeur $V_m$, la mesure du produit IxS du courant I anodique débité par le tube pendant la durée S de la pose et la mesure intégrée M du rayonnement ayant traversé l'étalon qui a été détecté par la cellule détectrice pendant le temps de pose S choisi pendant l'opération (d').

Dans le cas où le système radiologique présente plusieurs paramètres de configuration possibles, le procédé comprend une opération supplémentaire qui consiste en :

(k) la réitération des opérations (e) ou (e') à (j) pour chaque valeur des paramètres de configuration.

Dans le cas où les paramètres de configuration peuvent être groupés en classes, les différentes opérations (e) ou (e') à (j) sont effectuées pour une configuration de référence de chaque classe C et l'on détermine pour chaque élément de la classe C un coefficient de proportionnalité rapporté à la configuration de référence en mesurant le rendement $D_i$ à une tension $V_m$ et pour une valeur donnée de la grandeur physique $A_p$ et en définissant comme coefficient de proportionnalité de la configuration le rapport entre cette mesure du rendement $D_i$ et le rendement mesuré dans des conditions analogues avec la configuration de référence. Afin d'affiner la précision de ce coefficient, la valeur retenue pourrait résulter de la moyenne des rapports de rendements tels qu'ils viennent d'être définis et mesurés dans diverses conditions radiologiques.

Dans de nombreux systèmes radiologiques, la grandeur physique $A_p$ sera l'épaisseur de l'étalon suivant le trajet du rayonnement X. Le procédé d'étalonnage selon l'invention permet donc de déterminer l'épaisseur $E_p$ d'un étalon si l'on connaît $V_m$ et $D_i$. Ainsi, si pour un objet placé à la place de l'étalon, on obtient les mêmes valeurs $V_m$ et $D_i$, on en déduira que son épaisseur équivalente par rapport à l'étalon est $E_p$. Il est possible d'exploiter cette valeur en la communiquant à l'opérateur au moyen d'un dispositif d'affichage convenable ou en l'enregistrant pour un usage ultérieur. Le procédé d'étalonnage décrit ci-dessus permet donc de mesurer l'épaisseur équivalente d'un objet à l'aide d'un système radiologique.

D'autres caractéristiques et avantages de la présente invention apparaîtront à la lecture de la description suivante de l'invention faite en relation avec les dessins joints dans lesquels :

- la figure 1 est un schéma fonctionnel d'un système radiologique qui permet de mettre en oeuvre le procédé d'étalonnage selon l'invention et,

- la figure 2 est un diagramme montrant des courbes obtenues en mettant en oeuvre le procédé d'étalonnage selon l'invention.

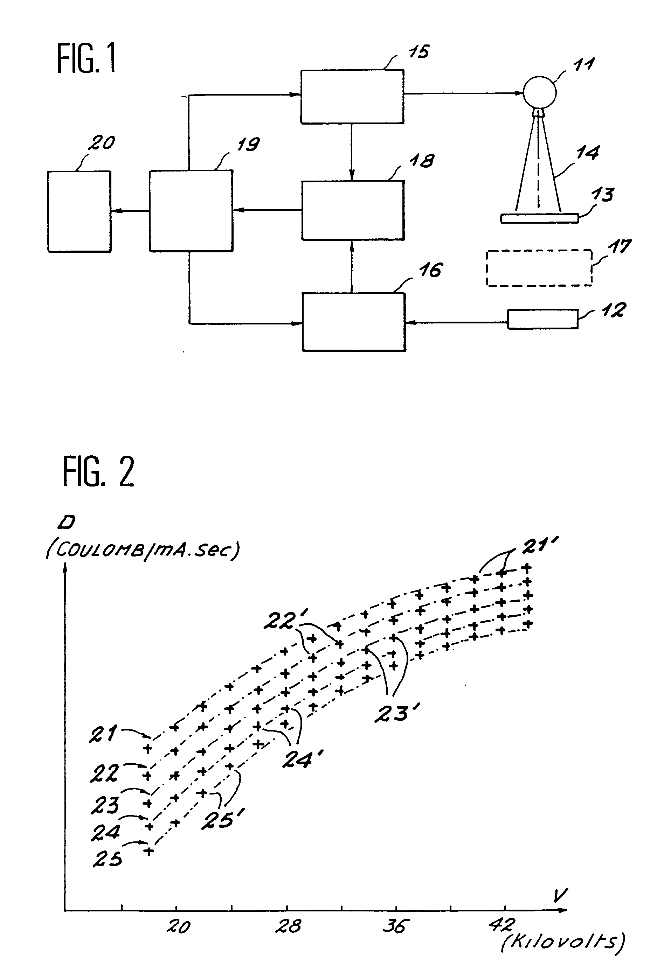

Un système radiologique auquel s'applique le procédé d'étalonnage selon l'invention comprend au moins une source 11 de rayonnement X et une cellule détectrice 12 du rayonnement X disposée en aval d'un objet 13 dans le sens du rayonnement X matérialisé, sur la figure 1, par un faisceau 14 de rayons X.

La source 11 est associée à un dispositif d'alimentation 15 qui fournit une haute tension d'alimentation variable $V_m$ pendant une durée variable S appelée durée de pose. Pendant la pose, le tube à rayons X de la source 11 est parcouru par un courant anodique I.

La cellule détectrice 12 permet de convertir une grandeur physique caractéristique du faisceau 14 de rayons X, tel que le KERMA ou la fluence énergétique, en un signal de mesure L, par exemple de type électrique. Le signal électrique L, fourni par la cellule détectrice 12, est appliqué à un circuit 16 qui réalise une intégration du signal électrique pendant la durée S de la pose. Le signal M, qui résulte de l'intégration, est une mesure du rayonnement ayant traversé l'objet 13 pendant la durée S de la pose.

Le système radiologique qui vient d'être décrit succinctement ne permet pas de réaliser une image de l'objet. Pour réaliser une telle image, il faut ajouter un récepteur 17, tel qu'un film sensible, qui peut être placé entre l'objet 13 et la cellule détectrice 12 ou en aval de cette dernière; dans une troisième variante du système radiologique, la cellule détectrice 12 peut être incorporée au récepteur 17.

Le procédé d'étalonnage consiste d'abord à choisir une classe d'objets de référence ou étalons et à effectuer des mesures de radiation, appelées également mesures de fonctionnement, pour chaque étalon et pour différentes valeurs $V_m$ de la tension d'alimentation de la source 11 de rayonnement X. Dans la classe d'objets de référence, la variable sera par exemple l'épaisseur $E_p$ de l'étalon qui intercepte perpendiculairement le rayonnement X. Cette épaisseur $E_p$ est la grandeur physique A qui caractérise l'objet à observer.

La mesure de radiation est le rendement D qui est défini comme le rapport entre la valeur M donnée par le circuit 16 et le produit IxS. En choisissant un tel rapport pour définir le rendement D, ce dernier est indépendant du temps de pose S et des différentes valeurs du courant du tube.

Le rendement D est calculé par un dispositif 18 qui reçoit le signal M du circuit 16 et l'information du produit IxS en provenance du dispositif d'alimentation 15.

Il va de soi que dans le cas où le courant I ne serait pas constant pendant la durée de la pose, le produit IxS serait remplacé par l'intégrale du courant anodique sur la durée de la pose.

De la même manière, il sera possible de mesurer le rendement D sur une fraction de la durée de la pose à condition que le signal M provienne d'une intégration du signal L sur la même durée que l'intégration du courant anodique I.

Ainsi, lorsque l'ensemble des étalons est déterminé, le procédé d'étalonnage consiste à mesurer le rendement pour chaque étalon aux tensions d'alimentation $V_m$ spécifiées. Plus précisément, avec un premier étalon d'épaisseur $E_1$, on effectue une mesure de rendement $D_{1m}$ pour chaque valeur $V_m$ de la tension d'alimentation, les différentes valeurs $V_m$ constituant un ensemble déterminé. Ces valeurs $D_{1m}$ en fonction de la tension $V_m$ peuvent être reportées sur un diagramme pour obtenir les points 21' de la figure 2.

Les mesures de rendement $D_i$ sont effectuées pour un autre étalon d'épaisseur $E_2$ et on obtient les valeurs $D_{2m}$ correspondant aux points 22' de la figure 3 et ainsi de suite pour obtenir les autres séries de points 23', 24' et 25' correspondant respectivement aux rendements $D_{3m}$ $D_{4m}$ et $D_{5m}$ et aux épaisseurs $E_3$, $E_4$ et $E_5$.

Il est à noter que sur la figure 2, les rendements $D_{pm}$ ont été reportés en ordonnées logarithmiques tandis que les tensions d'alimentation ont été reportées en abscisses de 20 kilovolts à 44 kilovolts.

Ces séries de points 21' à 25' servent à définir les paramètres d'un modèle analytique qui décrit le comportement du rendement $D_i$ en fonction des paramètres $V_m$ et $E_p$ pour une configuration donnée du système radiologique. Ce modèle analytique sera noté :

$$D_i = f (V_m, E_p) \qquad\qquad (1)$$

Les paramètres du modèle analytique peuvent être ajustés à l'aide d'outils classiques d'estimation tels que la méthode de minimisation de l'erreur quadratique.

Les courbes 21 à 25 représentent la valeur du rendement $D_i$ donnée par le modèle analytique représenté par l'expression :

$$D_i = f (V_m, E_p) = \exp [f_1 (V_m) + E_p \times f_2 (V_m)] \qquad\qquad (2)$$

dans laquelle $f_1 (V_m)$ et $f_2 (V_m)$ sont des polynômes du deuxième degré dont l'expression est donnée par :

$$f_1 (V_m) = A_O + A_1 V_m + A_2 V_m^2$$

$$f_2 (V_m) = B_O + B_1 V_m + B_2 V_m^2$$

La fonction inverse de celle exprimée par la formule (2) permet de calculer $E_p$ si l'on connaît $D_i$ et $V_m$ en utilisant la formule (3) suivante :

4

$$E_p = g\ (V_m, D) = \frac{Ln\ (D_i)\ -\ f_1\ (V_m)}{f_2\ (V_m)} \qquad (3)$$

sachant que $f_2\ (V_m)$ ne peut pas s'annuler pour les valeurs courantes de $V_m$ car le rendement $D_i$ dépend toujours de l'épaisseur Ep aux tensions $V_m$ considérées. En d'autres termes, à un couple de valeurs ($E_p$, $V_m$) correspond une mesure de rendement $D_i$, ce qui permet de déterminer $E_p$ en fonction de $V_m$ et D. Au cours d'un examen radiologique, une mesure de rendement $D_i$ qui est effectuée avec une tension d'alimentation $V_m$ donnée permet de déterminer une épaisseur équivalente exprimée dans les unités utilisées pour $E_p$.

Une application préférentielle sera le cas des examens mammographiques étant donné la faible variation de la composition des tissus mammaires.

En résumé, le procédé d'étalonnage du système radiologique consiste à effectuer les opérations suivantes :

(a) le choix d'une grandeur physique A qui caractérise l'objet à observer, par exemple l'épaisseur $E_p$ de l'objet,

(b) le choix de n objets de référence ou étalons dont la grandeur $A_p$ (l'épaisseur $E_p$) est différente d'un étalon au suivant,

(c) le choix de j valeurs $V_m$ de la tension (circuit 15) d'alimentation du tube à rayons X pour lesquelles l'étalonnage sera effectué,

(d) le choix de la valeur du produit IxS du courant I débité par le tube à rayons X pendant la durée S de la pose pour chaque étalon associé à chaque valeur de la tension $V_m$,

(e) la mise en place d'un étalon, le réglage de la tension à une valeur $V_m$, et la mesure de M (circuit 16) lorsque le produit IxS est égal à la valeur choisie pendant l'opération (d),

(f) le calcul du rendement D = M/IxS, dans le dispositif 18, ce qui donne un des points d'une des courbes de la figure 2.

Le calcul du rendement $D_i$ est effectué pour toutes les j valeurs de la tension $V_m$ sans changer l'étalon de place, c'est-à-dire en

(g) réitérant les opérations (e) et (f) pour un même étalon mais pour les (j-1) autres valeurs de la tension d'alimentation $V_m$.

On obtient ainsi un ensemble de points représenté, par exemple, par les points 21' de la figure 2. Pour obtenir le réseau de points complet (22' à 25') sur la figure 2, il faut :

(h) réitérer les opérations (e), (f) et (g) pour les (n-1) autres étalons.

De manière pratique, les n valeurs de $A_p$ (épaisseur $E_p$), les j valeurs de la tension d'alimentation $V_m$ et les (nxj) valeurs de $D_i$ sont fournies à un microprocesseur 19 qui, selon un logiciel approprié, réalise :

(i) la détermination du modèle analytique

$D_i = f\ (V_m, A)$ reliant les valeurs du rendement $D_i$ à celles de la grandeur physique $A_p$ (épaisseur $E_p$) et de la tension $V_m$ conformément à une méthode classique d'estimation.

Enfin, le microprocesseur 19 calcule la fonction inverse de $f\ (V_m, A_p)$ qui permet de déterminer $A_p$ - (épaisseur $E_p$) en fonction de $V_m$ et $D_i$. Cette fonction est notée $g\ (V_m, D_i)$.Dans une variante du procédé d'étalonnage, l'opération (d) peut être remplacée par une opération (d') qui consiste à choisir un temps de pose S pour chaque étalon associé à chaque valeur de la tension $V_m$. Dans ce cas, l'opération (e) est modifiée pour mesurer M et le produit IxS pendant le temps de pose S choisi pendant l'opération (d').

Dans le procédé d'étalonnage qui vient d'être décrit, on a supposé que seuls l'épaisseur de l'étalon et les paramètres radiologiques du système changeaient, les autres paramètres dits de configuration, tels que le filtre, restant identiques. On comprend alors que le procédé d'étalonnage doit être répété en changeant un seul des paramètres de configuration, par exemple le filtre. On obtient alors éventuellement un autre modèle analytique qui aboutira à une autre formulation de l'épaisseur $E_p$ en fonction de $D_i$ et $V_m$.

Comme les paramètres de configuration qui peuvent entrer en jeu sont nombreux, une telle manière de faire conduit à de nombreuses manipulations. Le nombre de ces manipulations d'étalonnage peut être réduit en remarquant qu'il existe une interdépendance entre certains de ces paramètres. Ces configurations forment des classes C qui ont la propriété suivante : les rendements $D_i$ qui sont mesurés, pour chacune des configurations définies par une classe C, se déduisent les uns des autres par un coefficient de proportionnalité pour des conditions radiologiques analogues. Ce facteur de proportionnalité traduit le fait que les spectres énergétiques incidents sur le détecteur sont, dans chacune des classes, semblables ou très proches.

Pour réduire le nombre d'étalonnages, l'invention propose de choisir, pour chaque classe C, une configuration de référence et d'effectuer un étalonnage complet tel que décrit ci-dessus. Il faut ensuite déterminer pour chaque élément de la classe C un coefficient de proportionnalité rapporté à la configuration de référence en mesurant le rendement $D_i$ à une tension $V_m$ et pour une valeur donnée de la grandeur physique $A_p$ et en définissant comme coefficient de proportionnalité de la configuration le rapport entre cette mesure du rendement $D_i$ et le rendement mesuré dans des conditions analogues avec la configuration de référence. Afin d'affiner la précision de ce coefficient, la valeur retenue pourrait résulter de la moyenne des rapports de rendements tels qu'ils viennent d'être définis et mesurés dans diverses conditions radiologiques. Cette moyenne peut être calculée à l'aide des rapports de rendements mesurés à plusieurs reprises pour le même élément de la classe C dans les mêmes conditions radiologiques. Elle peut également être calculée à l'aide des rapports de rendements mesurés, à une ou plusieurs reprises, avec divers étalons et pour diverses valeurs de la tension $V_m$.

Par ailleurs, si la fonction $f(V_m, A_p)$ est supposée séparable en deux fonctions, il est possible de réduire le nombre des mesures d'étalonnage en mesurant les rendements $D_i$ pour différentes valeurs $V_m$ de la tension d'alimentation, $E_p$ étant fixée, puis les rendements $D_i$ pour différentes valeurs $E_p$ de l'épaisseur, $V_m$ étant fixée. On obtient dans le premier cas la fonction :

$$G_{E_p}(V_m) = f(V_m, E_p)$$

pour $E_p$ fixée et dans le deuxième cas :

$$H_{V_m}(E_p) = f(V_m, E_p)$$

pour $V_m$ fixée Ainsi, dans le cas où la fonction $f(V_m, E_p)$ est séparable en deux fonctions, on obtient :

$$f(V_m, E_p) = G_{E_p}(V_m) \times H_{V_m}(E_p)$$

Les fonctions

$$G_{E_p}(V_m) \ et \ H_{V_m}(E_p)$$

peuvent être définies analytiquement de la manière suivante. On détermine d'abord, pour chacune de ces fonctions, le degré du polynôme propre à décrire la courbe et on détermine ensuite les coefficients de ce polynôme à l'aide d'une méthode d'estimation.

Pour mettre en oeuvre cette méthode, il faut $(n + j)$ mesures alors qu'il en faut $(n \times j)$ suivant la méthode de la figure 2.

L'invention a été décrite en utilisant des étalons d'épaisseur déterminée $E_p$ mais il est clair que les étalons peuvent être quelconques et, notamment, présenter des formes autres que parallélépidédiques, par exemple cylindriques.

Une fois étalonné de la manière décrite ci-dessus, le système radiologique est fonctionnel pour mesurer l'épaisseur équivalente d'un objet. Cette mesure se déroule selon les étapes suivantes :

(1) la mise en place de l'objet sur le trajet du rayonnement

(2) le réglage de la tension $V_m$ du tube à l'aide du dispositif d'alimentation 15,

(3) la mesure du rendement $D_i$ à l'aide du dispositif 18 qui reçoit les informations des valeurs $V_m$, I et S du dispositif 15 et l'information de mesure de rayonnement M du circuit 16,

(4) le calcul de l'épaisseur équivalente $E_p$ suivant la formule $E_p = g(V_m, D_i)$ à l'aide du microprocesseur 19,

(5) l'affichage du résultat du calcul sur un dispositif d'affichage 20.

Le dispositif d'affichage (20) peut être remplacé par tous autres moyens d'exploitation de l'épaisseur $E_p$ tels qu'un fichier informatique, une imprimante, un dispositif de calcul.

**Revendications**

1. Procédé d'étalonnage d'un système de radiologie prévu pour examiner un objet (13), ledit système comprenant :

- un tube (11) à rayons X dont la tension d'alimentation V peut prendre diverses valeurs $V_m$, à variation continue ou discrète, et qui émet un faisceau (14) de rayons X sous forme d'impulsions de durée variable S, et
- une seule cellule détectrice (12) des rayons X ayant traversé l'objet à examiner qui permet de convertir une grandeur physique caractérisant le faisceau de rayons X en un signal de mesure M tel qu'un signal électrique,

le procédé comprenant les opérations suivantes :

(a) le choix d'une grandeur physique A qui caractérise l'objet à observer,

(b) le choix d'une classe d'objets de référence qui comporte n objets ou étalons dont la grandeur physique A peut prendre n valeurs connues $A_p$,

(c) le choix de j valeurs $V_m$ de la tension d'alimentation du tube (11) à rayons X pour lesquelles l'étalonnage sera effectué,

(d) le choix de la valeur du produit IxS du courant anodique I débité par le tube (11) à rayons X pendant la durée S de la pose pour chaque étalon associé à chaque valeur de la tension d'alimentation $V_m$,

(e) la mise en place d'un étalon sur le trajet du rayonnement X, le réglage de la tension du tube (11) à une valeur $V_m$ et la mesure intégrée M du rayonnement (16) ayant traversé l'étalon, qui a été détecté par la cellule détectrice (12) entre le début de la mesure et l'instant où le produit IxS est égal à la valeur choisie pendant l'opération (d),

(f) le calcul (18) du rendement D donné par le rapport M/IxS,

(g) la réitération des opérations (e) et (f) pour le même étalon mais pour les (j-1) autres valeurs de la tension d'alimentation $V_m$,

(h) la réitération des opérations (e), (f) et (g) pour les (n-1) autres objets ou étalons,

(i) la détermination du modèle analytique $D_i = f(V_m, A_p)$ reliant les (nxj) valeurs du rendement $D_i$ à celles de la grandeur physique $A_p$ et de la tension $V_m$ et,

(j) la détermination de la fonction inverse de $f(V_m, A_p)$, notée $g(V_m, D_i)$, qui permet de déterminer A en connaissant $V_m$ et $D_i$.

2. Procédé d'étalonnage d'un système de radiologie prévu pour examiner un objet (13), ledit système comprenant :

- un tube (11) à rayons X dont la tension d'alimentation V peut prendre diverses valeurs $V_m$, à variation continue ou discrète, et qui émet un faisceau (14) de rayons X sous forme d'impulsions de durée variable S, et
- une seule cellule détectrice (12) des rayons X ayant traversé l'objet à examiner qui permet de convertir une grandeur physique caractérisant le faisceau de rayons X en un signal de mesure M tel qu'un signal électrique,

le procédé comprenant les opérations suivantes :

(a) le choix d'une grandeur physique A qui caractérise l'objet à observer,

(b) le choix d'une classe d'objets de référence qui comporte n objets ou étalons dont la grandeur physique A peut prendre n valeurs connues $A_p$,

(c) le choix de j valeurs $V_m$ de la tension d'alimentation du tube (11) à rayons X pour lesquelles l'étalonnage sera effectué,

(d') le choix du temps de pose S pour chaque étalon associé à chaque valeur de la tension $V_m$,

(e') la mise en place d'un étalon sur le trajet du rayonnement X, le réglage de la tension du tube à une valeur $V_m$, la mesure du produit IxS du courant I anodique débité par le tube (11) à rayons X pendant la durée S de la pose et la mesure intégrée M du rayonnement ayant traversé l'étalon qui a été détecté par la cellule détectrice pendant le temps de pose S choisi pendant l'opération (d').

(f) le calcul (18) du rendement D donné par le rapport M/IxS,

(g) la réitération des opérations (e) et (f) pour le même étalon mais pour les (j-1) autres valeurs de la tension d'alimentation $V_m$,

(h) la réitération des opérations (e), (f) et (g) pour les (n-1) autres objets ou étalons,

(i) la détermination du modèle analytique $D_i = f(V_m, A_p)$, reliant les (nxj) valeurs du rendement $D_i$ à celles de la grandeur physique $A_p$ et de la tension $V_m$, et

(j) la détermination de la fonction inverse de $f(V_m, A_p)$, notée $g(V_m, D_i)$, qui permet de déterminer A en connaissant $V_m$ et $D_i$.

3. Procédé d'étalonnage selon la revendication 1, ou 2 caractérisé en ce que la fonction $f(V_m, A)$ est supposée séparable en deux fonctions, $G_{A_p}(V_m)$ et $H_{V_m}(A_p)$, et en ce que les opérations suivantes sont effectuées:

(m) la détermination d'un premier modèle analytique

$$G_{A_p}(V_m)$$

reliant les valeurs de rendement $D_i$ à celles de la tension d'alimentation $V_m$ pour $A_p$ fixé,

(n) la détermination d'un deuxième modèle analytique $H_{V_m}(A_p)$ reliant les valeurs de rendement $D_i$ à celles de la grande physique $A_p$ pour $V_m$ fixé, et

(o) la détermination d'un modèle analytique $D_i = f(V_m, A_p)$ résultant de la multiplication des fonctions $G_A(V_m)$ et $H_{V_m}(A_p)$,

ce qui permet de réduire le nombre des mesures d'étalonnage.

4. Procédé d'étalonnage selon la revendication 1, 2 ou 3 dans le cas où le système de radiologie présente plusieurs paramètres de configuration, caractérisé en ce qu'il comprend en outre une opération qui consiste en : (k) la réitération des opérations (e) à (j) pour chaque valeur des paramètres de configuration.

5. Procédé d'étalonnage selon la revendication 4, caractérisé en ce que les paramètres sont groupés en classes, en ce que les différentes opérations (e) à (j) sont effectuées pour une configuration de référence de chaque classe (C) et en ce que l'on détermine, pour chaque élément de la classe C, un coefficient de proportionnalité rapporté à la configuration de référence en mesurant le rendement $D_i$ à une tension $V_m$ et pour une valeur donnée de la grandeur physique $A_p$ et en définissant comme coefficient de proportionnalité de la configuration le rapport entre cette mesure du rendement $D_i$ et le rendement mesuré dans des conditions analogues avec la configuration de référence.

6. Procédé d'étalonnage selon la revendication 5, caractérisé en ce que l'on calcule la moyenne des rapports de rendements mesurés à plusieurs reprises pour le même élément de la classe C dans les mêmes conditions radiologiques pour obtenir le coefficient de proportionnalité.

7. Procédé d'étalonnage selon la revendication 5, caractérisé en ce que l'on calcule la moyenne des rapports de rendements mesurés, à une ou plusieurs reprises, pour le même élément de la classe C avec divers étalons et pour diverses valeurs de la tension $V_m$ pour obtenir le coefficient de proportionnalité.

8. Procédé d'étalonnage selon l'une quelconque des revendications 1 à 7, caractérisé en ce que la grandeur physique $A_p$ est l'épaisseur $E_p$ de l'étalon dans la direction du rayonnement X.

9. Procédé d'étalonnage selon la revendication 8, caractérisé en ce que le modèle analytique s'écrit sous la forme :

$$f(V_m, E_p) = \exp[f_1(V_m) + E_p \times f_2(V_m)]$$

dans laquelle $f_1(V_m)$ et $f_2(V_m)$ sont des polynômes du deuxième degré dont l'expression est donnée par :

$$f_1(V_m) = A_0 + A_1 V_m + A_2 V_m^2$$

$$f_2(V_m) = B_0 + B_1 V_m + B_2 V_m^2$$

8

**10.** Procédé d'étalonnage selon la revendication 9, caractérisé en ce que la fonction inverse g $(V_m,D_i)$ qui relie la mesure $D_i$ et la tension d'alimentation $V_m$ à l'épaisseur $E_p$ est donnée par l'expression :

$$g\ (V_m,D_i)\ =\ E_p\ =\ \frac{Ln\ (D_i)\ -\ f_1\ (V_m)}{f_2\ (V_m)}$$

**11.** Procédé de mesure de l'épaisseur équivalente $E_p$ d'unobjet à l'aide d'un système de radiologie étalonné selon le procédé de l'une quelconque des revendications 1 à 10, et dans lequel la grandeur physique $A_p$ est définie comme l'épaisseur équivalente $E_p$, caractérisé en ce le calcul de l'épaisseur équivalente $E_p$ est réalisé à l'aide de la formule $E_p = g(V_m, D_i)$.

**12.** Procédé selon la revendication 11, caractérisé en qu'il comprend, en outre, l'opération d'affichage (20) de l'épaisseur équivalente calculée $E_p$.

**Claims**

**1.** A calibration method for a radiology system for examining an object (13), this system comprising:
- an X-ray tube (11) whose supply voltage V can assume different values $V_m$, varying continuously or discretely, and which emits a beam (14) of X-rays in the form of pulses of variable duration S, and
- a single cell for detecting (12) the X-rays that have passed through the object under examination to enable a physical size that is characteristic of the X-ray beam to be converted into a measurement signal M such as an electric signal,

the method comprising the following operations:
(a) selecting a physical size A which characterises the object under observation,
(b) selecting a category of reference objects comprising n objects or gauges whose physical size A may be n known values $A_p$,
(c) selecting j supply voltage values $V_m$ of the X-ray tube (11) for which calibration will be carried out,
(d) selecting the value of the product of IxS of the anode current I delivered by the X-ray tube (11) over the exposure duration S for each gauge associated with each supply voltage value $V_m$,
(e) positioning a gauge in the path of the X-ray, setting the voltage of the tube (11) to a value $V_m$ and taking the integrated measurement M of the radiation (16) that has passed through the gauge and has been picked up by the detector cell (12) between commencement of the measuring process and the instant at which the product of IxS is equal to the value selected during operation (d),
(f) calculating (18) the product D given by the ratio M/IxS,
(g) repeating operations (e) and (f) for the same gauge but for the other (j-1) values of the supply voltage $V_m$,
(h) repeating operations (e), (f) and (g) for the other (n-1) objects or gauges,
(i) determining the analytical model $D_i = f(V_m,A_p)$ linking the (nxj) product values $D_i$ to those of physical size $A_p$ and voltage $V_m$, and
(j) determining the inverse function of $f(V_m,A_p)$, expressed as $g(V_m,D_i)$, which makes it possible to determine A where $V_m$ and $D_i$ are known.

**2.** A calibration method for a radiology system for examining an object (13), this system comprising:
- an X-ray tube (11) whose supply voltage V may assume different values $V_m$, varying continuously or discretely, and which emits a beam of X-rays (14) in the form of pulses of variable duration S, and
- a single cell for detecting (12) the X-rays that have passed through the object to be examined which makes it possible to convert a physical size that is characteristic of the X-ray beam into a measurement signal M such as an electrical signal,

the method comprising the following operations:
(a) selecting a physical size A which characterises the object under observation,
(b) selecting a category of reference objects which comprises n objects or gauges whose physical size A may be n known values $A_p$,

(c) selecting j supply voltage values $V_m$ of the X-ray tube (11) at which calibration will be carried out,

(d') selecting the exposure duration S for each gauge associated with each voltage value $V_m$,

(e') placing a gauge in the path of the X-ray, setting the voltage of the tube to a value $V_m$, measuring the product of IxS of the anode current I delivered by the X-ray tube (11) over the exposure duration S and taking the integrated measurement M of the radiation that has passed through the gauge and been picked up by the detector cell over the exposure duration S selected during operation (d'),

(f) calculating (18) the product D given by the ratio M/IxS,

(g) repeating operations (e) and (f) for the same gauge but for the other (j-1) values of supply voltage $V_m$,

(h) repeating operations (e), (g) and (g) for the other (n-1) objects or gauges,

(i) determining the analytical model $D_i$-f($V_m$,$A_p$), linking the (nxj) values of the product $D_i$ to those of physical size $A_p$ and voltage $V_m$, and

(j) determining the inverse function of f($V_m$,$A_p$), expressed as g($V_m$, $D_i$), which makes it possible to determine A where $V_m$ and $D_i$ are known.

3. Calibration method as claimed in claim 1 or 2, characterised in that it is assumed that the function f ($V_m$, A) can be separated into two functions, $G_{Ap}(V_m)$ and $H_{Vm}(A_p)$, and in that the following operations are carried out:

(m) determining a first analytical model $G_{Ap}(V_m)$ linking the values of the product $D_i$ to those of supply voltage $V_m$ for a fixed $A_p$ value,

(n) determining a second analytical model $H_{Vm}(A_p)$ linking the values of the product $D_i$ to those of physical size $A_p$ for a fixed $V_m$ value, and

(o) determining an analytical model $D_i = f(V_m,A_p)$ which is the product of multiplying the functions $G_A$-($V_m$) and $H_{Vm}(A_p)$,

making it possible to reduce the number of calibration measurements.

4. A calibration method as claimed in claims 1, 2 or 3, where the radiology system has several configuration parameters, characterised in that it includes in addition an operation consisting in:

(k) repeating operations (e) to (j) for each value of the configuration parameters.

5. A calibration method as claimed in claim 4, characterised in that the parameters are grouped in categories, in that the various operations (e) to (j) are carried out for a reference configuration of each category (c) and in that for each element in the category (C) a coefficient of proportionality relative to the reference coefficient is determined by measuring the product $D_i$ at a voltage $V_m$ and for a given value of physical size $A_p$ and by calculating the ratio between this measurement of the product $D_i$ and the product measured under similar conditions using the reference configuration in order to obtain the proportionality coefficient of the configuration.

6. A calibration method as claimed in claim 5, characterised in that the average of the ratios of products measured at certain points in time for the same element of the category C under the same radiological conditions is calculated in order to obtain the coefficient of proportionality.

7. A calibration method as claimed in claim 5, characterised in that the average of the ratios of products measured, at one or several point(s) in time, for the same element in the category C with various gauges and for various voltage values $V_m$ is calculated in order to obtain the coefficient of proportionality.

8. A calibration method as claimed in any one of claims 1 to 7, characterised in that the physical size $A_p$ is the thickness $E_p$ of the gauge in the direction of the X-ray.

9. A calibration method as claimed in claim 8, characterised in that the analytical model is expressed in the form:

$$f(V_m, E_p) = \exp[f_1(V_m) + E_p \times f_2(V_m)]$$

in which $f_1(V_m)$ and $f_2(V_m)$ are polynomials of the second degree expressed by: ) are polynomials of the second degree expressed by:

$$f1\ (V_m)\ =\ A_o\ +\ A_1\ V_m\ +\ A_2\ V^2_m$$
$$f_2\ (V_m)\ =\ B_o\ +\ B_1\ V_m\ +\ B_2\ V^2_m$$

**10.** A calibration method as claimed in claim 9, characterised in that the inverse function $g(V_m,D_i)$ which links the measurement $D_i$ and the supply voltage $V_m$ to the thickness $E_p$ is given by the expression:

$$g(V_m,D_i) = E_p = \frac{Ln(D_i) - f_1(V_m)}{f_2(V_m)}$$

**11.** A method of measuring the equivalent thickness $E_p$ of an object using a radiology system calibrated by the method of any one of claims 1 to 10, and in which the physical size $A_p$ is defined as being the equivalent thickness $E_p$, characterised in that the equivalent thickness $E_p$ is calculated by using the formula $E_p = g(V_m,D_i)$.

**12.** A method as claimed in claim 11, characterised in that it also incorporates an indicator display (20) of the equivalent thickness $E_p$ calculated.

**Patentansprüche**

**1.** Verfahren zum Eichen eines zur Untersuchung eines Gegenstandes (13) vorgesehenen Röntgensystems mit:
- einer Röntgenröhre (11), deren Versorgungsspannung V verschiedene Werte $V_m$ annehmen kann, die kontinuierlich oder stufenweise variabel sind und die einen Röntgenstrahl (14) in Form von Impulsen variabler Dauer S aussendet und mit
- einer einzigen Detektorzelle (12) für die Röntgenstrahlen, welche den zu untersuchenden Gegenstand durchsetzt haben und die eine den Röntgenstrahl charakterisierende physikalische Größe in ein Meßsignal M, z.B. ein elektrisches Signal, umwandelt, wobei das Verfahren die folgenden Schritte aufweist:

(a) die Wahl einer physikalischen Größe A, welche den zu beobachtenden Gegenstand charakterisiert,

(b) die Wahl einer Klasse von Referenzgegenständen, welche n Gegenstände oder Muster aufweist, deren physikalische Größe A n bekannte Werte $A_p$ annehmen kann,

(c) die Wahl von j Werten $V_m$ der Versorgungsspannung der Röntgenröhre (11), für welche die Eichung durchgeführt wird,

(d) die Wahl des Wertes des Produktes IxS des Anodenstromes I der Röntgenröhre (11) während der Dauer S der Aufnahme für jedes Muster, welches jedem Wert der Versorgungsspannung $V_m$ zugeordnet ist,

(e) das Anordnen eines Musters auf dem Weg der Röntgenstrahlung, die Regelung der Spannung der Röhre (11) auf einen Wert $V_m$ und die integrierte Messung M der Strahlung (16) nach dem Durchsetzen des Musters, welche von der Meßzelle (12) zwischen dem Beginn der Messung und demjenigen Zeitpunkt gemessen worden ist, zu dem das Produkt IxS gleich dem gewählten Wert während des Schrittes (d) ist,

(f) die Berechnung (18) des durch das Verhältnis M/IxS erhaltenen Wirkungsgrades D,

(g) die Wiederholung der Schritte (e) und (f) für das gleiche Muster, jedoch für (j-1) andere Werte der Versorgungsspannung $V_m$,

(h) die Wiederholung der Schritte (e), (f) und (g) für die (n-1) anderen Gegenstände oder Muster,

(i) die Bestimmung des analytischen Modells $D_i = f(V_m,A_p)$, welches die (nxj) Werte des Wirkungsgrades $D_i$ mit denjenigen der physikalischen Größe $A_p$ und der Spannung $V_m$ verbindet und

(j) die Bestimmung der inversen Funktion von $f(V_m,A_p)$, benannt $g(V_m,D_i)$ zur Bestimmung von A bei Kenntnis von $V_m$ und $D_i$.

**2.** Verfahren zum Eichen eines zur Untersuchung eines Gegenstandes (13) vorgesehenen Röntgensystems mit:

- einer Röntgenröhre (11), deren Versorgungsspannung V verschiedene Werte $V_m$ annehmen kann, die kontinuierlich oder stufenweise variabel sind und die einen Röntgenstrahl (14) in Form von Impulsen variabler Dauer S aussendet und mit

- einer einzigen Detektorzelle (12) für die Röntgenstrahlen, welche den zu untersuchenden Gegenstand durchsetzt haben und die eine physikalische Größe, welche den Röntgenstrahl charakterisiert, in ein Meßsignal M, wie z.B. ein elektrisches Signal umwandelt, wobei das Verfahren die folgenden Schritte aufweist:

(a) die Wahl einer physikalischen Größe A, welche den zu beobachtenden Gegenstand charakterisiert,

(b) die Wahl einer Klasse von Referenzgegenständen, welche n Gegenstände oder Muster enthält, deren physikalische Größe A n bekannte Werte $A_p$ annehmen kann,

(c) die Wahl von j Werten $V_m$ für die Versorgungsspannung der Röntgenröhre (11), für welche die Eichung durchgeführt wird,

(d') die Wahl der Belichtungszeit S für jedes Muster, das jedem Wert der Spannung $V_m$ zugeordnet ist,

(e') das Anordnen eines Musters im Weg der Röntgenstrahlung, die Regelung der Spannung der Röhre auf einen Wert $V_m$, die Messung des Produktes IxS des Anodenstromes der Röntgenröhre (11) während der Dauer S der Belichtung und die integrierte Messung M der Strahlung, die das Muster durchsetzt hat und die von der Meßzelle während der Belichtungszeit S gemessen worden ist, welche während des Schrittes (d') ausgewählt wurde.

(f) die Berechnung (18) des durch das Verhältnis M/IxS gegebenen Wirkungsgrades D,

(g) die Wiederholung der Schritte (e) und (f) für das gleiche Muster, jedoch für die (j-1) anderen Werte der Versorgungsspannung $V_m$,

(h) die Wiederholung der Schritte (e), (f) und (g) für die (n-1) anderen Gegenstände oder Muster,

(i) die Bestimmung des analytischen Modells $D_i = f(V_m, A_p)$, welches die (nxj) Werte des Wirkungsgrades $D_i$ mit denjenigen der physikalischen Größe $A_p$ und der Spannung $V_m$ verbindet und

(j) die Bestimmung der inversen Funktion von $f(V_m, A_p)$ auch $g(V_m, D_i)$ genannt, um so A bei Kenntnis von $V_m$ und $D_i$ zu bestimmen.

3. Verfahren zum Eichen nach Anspruch 1 oder 2, dadurch gekennzeichnet, daß die Funktion f $(V_m, A)$ als in Funktionen $G_{Ap}(V_m)$ und $H_{Vm}(A_p)$ zerlegbar angesehen wird und daß die folgenden Schritte ausgeführt werden:

(m) die Bestimmung eines ersten analytischen Modells $G_{Ap}(V_m)$, welche die Werte des Wirkungsgrades $D_i$ mit denjenigen der Versorgungsspannung $V_m$ für einen feststehenden Wert $A_p$ verbindet,

(n) die Bestimmung eines zweiten analytischen Modells $H_{Vm}(A_p)$, welches die Werte für den Wirkungsgrad $D_i$ mit denjenigen der physikalischen Größe $A_p$ für einen feststehenden Wert $V_m$ verbindet und

(o) die Bestimmung eines analytischen Modells $D_i = f(V_m, A_p)$ resultierend aus der Multiplikation der Funktionen $G_A (V_m)$ und $H_{Vm} (A_p)$,

wodurch die Anzahl der Eichmessungen verringert wird.

4. Verfahren zum Eichen nach Anspruch 1, 2 oder 3, in dem Fall, in dem das Röntgensystem mehrere Konfigurationsparameter aufweist, dadurch gekennzeichnet, daß es außerdem einen Schritt aufweist, der besteht aus: (k) der Wiederholung der Schritte (e) und (j) für jeden Werte der Konfigurationsparameter.

5. Verfahren zum Eichen nach Anspruch 4, dadurch gekennzeichnet, daß die Parameter in Klassen zusammengefaßt sind und daß die verschiedenen Schritte (e) bis (j) durchgeführt werden für eine Referenzkonfiguration für eine jede Klasse C und daß für jedes Teil der Klasse C ein Proportionalitätskoeffizient bestimmt wird, der der Referenzkonfiguration zugeordnet wird, indem der Wirkungsgrad $D_i$ für eine Spannung $V_m$ und für einen gegebenen Wert der physikalischen Größe $A_p$ gemessen wird und indem als Proportionalitätskoeffizient für die Konfiguration das Verhältnis zwischen dieser Messung des Wirkungsgrades $D_i$ und demjenigen Wirkungsgrad festgelegt wird, welcher unter analogen Bedingungen zu der Referenzkonfiguration gemessen wird.

6. Verfahren zum Eichen nach Anspruch 5, dadurch gekennzeichnet, daß der Mittelwert der Verhältnisse der bei mehreren Wiederholungen für das gleiche Element der Klasse C, bei den gleichen radiologischen Bedingungen, gemessenen Wirkungsgrade berechnet wird, zum Erhalt des Proportionalitätskoeffizienten.

**7.** Verfahren zum Eichen nach Anspruch 5, dadurch gekennzeichnet, daß der Mittelwert der Verhältnisse der gemessenen Wirkungsgrade berechnet wird, bei einer oder mehreren Wiederholungen, für das gleiche Teil der Klasse C mit verschiedenen Mustern und für verschiedene Spannungswerte $V_m$, zum Erhalt des Proportionalitätskoeffizienten.

**8.** Verfahren zum Eichen nach einem der Ansprüche 1 bis 7, dadurch gekennzeichnet, daß die physikalische Größe $A_p$ die Dicke des Musters $E_p$ in Richtung der Röntgenstrahlung ist.

**9.** Verfahren zum Eichen nach Anspruch 8, dadurch gekennzeichnet, daß das analytische Modell die folgende Form aufweist:

$$f\,(V_m,\,E_p)\ =\ \exp\,[f_1\,(V_m)\ +\ E_p\ x\ f_2\,(V_m)]$$

wobei $f_1\,(V_m)$ und $f_2\,(V_m)$ Polynome der zweiten Art sind, die sich wie folgt ausdrücken lassen: ) Polynome der zweiten Art sind, die sich wie folgt ausdrücken lassen:

$$f_1\,(V_m)\ =\ A_o\ +\ A_1\ V_m\ +_m A_2\ V^2$$
$$f_2\,(V_m)\ =\ B_o\ +\ B_1\ V_m\ +_m B_2\ V^2$$

**10.** Verfahren zum Eichen nach Anspruch 9, dadurch gekennzeichnet, daß die inverse Funktion $g\,(V_m, D_i)$, welche die Messung $D_i$ und die Versorgungsspannung $V_m$ mit der Dicke $E_p$ verbindet durch den folgenden Ausdruck darstellbar ist:

$$g\,(V_m, D_i)\ =\ E_p\ =\ \frac{Ln\ (D_i)\ -\ f_1\,(V_m)}{f_2\,(V_m)}$$

**11.** Verfahren zur Messung der äquivalenten Dicke $E_p$ eines Gegenstandes mit Hilfe eines nach dem Verfahren 1 bis 10 geeichten Röntgensystems, bei dem die physikalische Größe $A_p$ als äquivalente Dicke $E_p$ definiert ist, dadurch gekennzeichnet, daß die Berechnung der äquivalenten Dicke $E_p$ mit Hilfe der Formel $E_p\ =\ g(V_m, D_i)$ erfolgt.

**12.** Verfahren nach Anspruch 11, dadurch gekennzeichnet, daß es außerdem den Schritt der Anzeige (20) der berechneten äquivalenten Dicke $E_p$ aufweist.

FIG.1

FIG. 2